# EUROPÄISCHE PATENTANMELDUNG

(11) **EP 1 956 570 A1**
(43) Veröffentlichungstag der Anmeldung: **13.08.2008**
(21) Anmeldenummer: 08100941.7
(22) Anmeldetag: 25.01.2008
(51) Int. Cl.: G08B 21/22, A61B 5/11

(54) **Verfahren zur zentralen Überwachung sowie Anordnung zur Aufnahme, Auswertung und selektiven Anzeige von Bildern ruhender Personen**

(30) Priorität: 09.02.2007 DE 102007006566
(71) Anmelder: SIEMENS AKTIENGESELLSCHAFT, 80333 München (DE)
(72) Erfinder: Doemens, Günter, 83607, Holzkirchen (DE); Laloni, Claudio, 82024, Taufkirchen (DE)

(57) **Zusammenfassung**

Verfahren zur zentralen Überwachung ruhender Personen bestehend aus folgenden Schritten:
- Fortlaufende Erfassung einer im infrarot beleuchteten oberen Oberfläche eines Bettes (2) mit darin ruhender Person (1) mittels eines dreidimensional auflösenden Sensors (5) und Ausgabe eines 3D-Bilds,
- fortlaufende Berechnung des Bettvolumens und dessen zeitlicher Veränderung auf der Basis des 3D-Bilds,
- Bewegungsanalyse der überwachten Person, wobei aufgrund einer Überschreitung mindestens eines festgelegten Grenzwerts bezüglich der Volumenänderung mindestens eine über dem Bett angeordnete Videokamera (11) aktiviert wird, und
- Signale der Videokamera (11) auf einen zentralen Videomonitor (9) geschaltet werden.

## Beschreibung

Die Erfindung betrifft ein Verfahren sowie eine Anordnung, womit mittels mindestens eines Sensors Personen hinsichtlich ihres Bewegungsverhaltens in Ruhephasen überwacht werden.

Es ist allgemein bekannt, dass in Alten- und Pflegeheimen sowie in Teilbereichen von Krankenhäusern die permanente Überwachung von Personen bzw. Patienten während der Nachtruhe oder allgemein während des Aufenthaltes in einem Bett aus Sicherheitsgründen notwendig ist. Durch die angespannte Kostensituation im Personalbereich ist dies jedoch nur sehr begrenzt realisierbar. Aus diesem Grund werden beispielsweise in Alten- und Pflegeheimen Stürze aus dem Bett oft nicht rechtzeitig wahrgenommen. Verbunden damit sind häufig ernsthafte Folgeverletzungen, weil eine erste Hilfe zu spät angewandt wird. Der Einsatz bestimmter Hilfsmittel zur Überwachung von Personen in Alten- und Pflegeheimen bzw. in Krankenhäusern bei geringem Personalaufwand und Investitionskosten wird weiterhin dadurch erschwert, dass Patient und Pflegepersonal die entsprechenden Mittel bei der täglichen Arbeit auch akzeptieren müssen.

Eine permanente Patientenüberwachung im Bett wird heutzutage in der Regel mit Druckmatten durchgeführt. Damit werden Belastungen bzw. Belastungsänderungen in entsprechende elektrische Signale umgewandelt und ausgegeben. Derartige Matten finden zum einen aus hygienischen Gründen wenig Akzeptanz, zum anderen liefert das elektrische Signal nur eine sehr eingeschränkte Information über die Situation und das Verhalten der ruhenden Person.

Übliche Auswertungen von zweidimensionalen Videobildern liefern nicht die notwendige Erkennungssicherheit und sind somit nicht für einen Dauereinsatz geeignet.

Der Erfindung liegt die Aufgabe zugrunde, mit einem Minimum an Betriebspersonal zentral eine erhebliche Anzahl von Betten permanent, sicher und mit nicht berührendem Verfahren zu überwachen.

Die Lösung dieser Aufgabe geschieht durch die jeweilige Merkmalskombination des Anspruchs 1 bzw. Anspruchs 8.

Vorteilhafte Ausgestaltungen können den Unteransprüchen entnommen werden.

Der Erfindung liegt die Erkenntnis zugrunde, dass einerseits der Einsatz von optischen Sensoren ein berührungsloses Verfahren gewährleistet, andererseits eine ständige und sichere Überwachung erzielbar ist, darüber hinaus kostengünstige Systeme bereitstehen und zusätzlich eine hohe Akzeptanz beispielsweise beim Pflegepersonal und beim Patienten erreichbar sind. Der Kerngedanke besteht dabei darin, dass aufgrund einer dreidimensionale Bewegungsanalyse im Bereich des Patientenbettes, von oben betrachtet, einer Kategorisierung der Bewegungsheftigkeit errechnet bzw. ermittelt wird und in besonderen Fällen eine Kamera zur Aufnahme eines Videobildes zugeschaltet wird, welches direkt, nachdem ein kritischer Zustand durch die Bewegungsanalyse ermittelt worden ist, auf einen zentralen Videomonitor aufgelegt wird.

Die Erfassung eines von oben beleuchteten und betrachteten Bettes mit darin ruhender Person geschieht demnach über einen dreidimensional auflösenden optischen Sensor, welcher ein 3D-Signal ausgibt. Ein derartiger Sensor liefert Konturbilder bzw. Höhenrasterbilder, die falls sie fortlaufend aufgenommen werden, Volumenveränderungen an der Oberfläche bei entsprechendem Vergleich zeitlich aufeinander folgender Bilder erkennen lassen.

Dreidimensional auflösende Sensoren können beispielsweise mit Hilfe des Triangulations-Prinzips dreidimensionale Oberflächen-Koordinaten aufnehmen, falls Beleuchtungsrichtung und Erfassungsrichtung unterschiedlich sind. Derartige Sensoren weisen eine strukturierte Beleuchtung auf.

Eine weitere Art von Sensoren zur Aufnahme von dreidimensionalen Oberflächen beruht auf Lichtlaufzeit-Messungen. Diese Sensoren weisen keinen Winkel zwischen Beleuchtungs- und Erfassungsrichtung auf.

Ist durch die fortlaufende Erfassung und Berechnung des Bettvolumens von oben eine Bewegungsanalyse ermittelt, so kann durch Vergleich mit bestimmten Sollwerten bezüglich der Volumenänderung ein für die entsprechende, in dem Bett ruhende Person, kritischer Zustand festgestellt werden. Dies löst automatisch die Aktivierung einer über dem Bett befindlichen Videokamera, die ebenfalls im Infrarotlicht arbeitet, aus. Mittels dieser, vorzugsweise im nahen Infrarotbereich betriebenen Videokamera kann ein Videobild der Oberfläche des Bettes aufgenommen und an einen zentralen Videomonitor weitergeleitet werden.

Zur Vermeidung nachträglicher Verkabelungen können Videokameras drahtlos Bilder an einen Monitor übermitteln.

Es ist vorteilhaft, eine einzige Videokamera einzusetzen, die sowohl eine optische Einheit für einen 3D-Sensor zur Aufnahme von dreidimensionalen Bildern bzw. dreidimensionalen Signalen darstellt, als auch die Videokamera mittels der nach entsprechender Bewegungsanalyse und Erkennung eines kritischen Zustands zugeschaltet wird und ein Videobild zur Weiterleitung an einen zentralen Monitor aufnimmt.

Das gesamte System arbeitet im infraroten Lichtwellenbereich, insbesondere im nahen Infrarotlicht. Viele Videokameras funktionieren in diesem Infrarotbereich, deren Geräte kostengünstig zur Verfügung sind.

Im Folgenden werden anhand von schematischen, die Erfindung nicht einschränkenden Figuren, Ausführungsbeispiele beschrieben:
- Figur 1: zeigt die Patientenüberwachung mittels optischer dreidimensionaler Sensorik, insbesondere anhand von Bewegungsanalyse,
- Figuren 2, 3 und 4: zeigen jeweils eine dreidimensionale Bewegungsanalyse, wobei Figur 2 eine Szene mit ruhiger, nicht bewegter Person wiedergibt,
- Figur 3: eine unruhige, leicht bewegte Person und
- Figur 4: eine erregte Person andeutet, die kräftige Bewegungen vollzieht.
- Figur 5: zeigt eine dezentrale Patientenüberwachung, wobei eine jeweilige Bewegungsanalyse dezentral durchgeführt wird und im Ereignisfall eine jeweils zugeschaltete Videokamera ein Bild an einen zentralen Überwachungsmonitor übermittelt.

Die mit der Erfindung verbundenen Vorteile liegen in einer wesentlich intensiveren und zuverlässigeren Patientenüberwachung ohne Erhöhung des Personalaufwands. Gleichzeitig ist eine größere Akzeptanz durch Patienten und Pflegepersonal zu erwarten. Dies gilt insbesondere für Alten- und Pflegeheime. Im privaten Bereich ist das Verfahren auch sehr gut für die Überwachung von Kinderbetten geeignet. Technisch wird dies durch eine permanente Bewegungsanalyse im dreidimensionalen Raum realisiert, die automatisch bei Überschreiten bestimmter Kriterien die Videokamera über einem Patientenbett auf einen zentralen Überwachungsmonitor durchschaltet. Die 3D-Bewegungsanalyse beruht auf der Erfassung des Volumens der von oben betrachteten Bettszene oder Teilbereichen davon bzw. auf der Volumenänderung über die Zeit und/oder dem Ort. Eine automatische Beobachtung der Volumenänderung stellt eine robuste und zuverlässige Basis für eine Bewegungsanalyse dar, anhand der dann die Videokamera auf eine zentrale Überwachungsstation geschaltet wird.

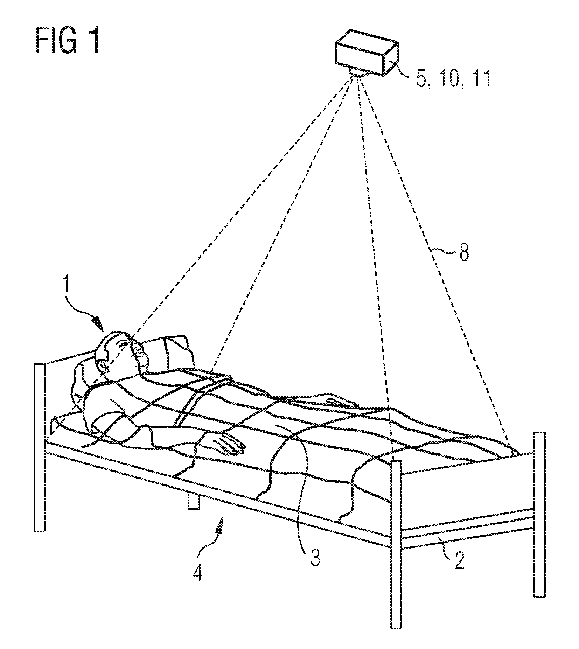

Figur 1 zeigt eine mit der Erfindung überwachbare Szene, bestehend aus einer Person 1, einem Bett 2, einer oberen Oberfläche 3, unter der ein bestimmtes Volumen vorhanden ist, eine insgesamt oder in Teilbereichen veränderliche Oberflächenkontur lässt direkt auf eine entsprechende Volumenänderung schließen. Voraussetzung ist natürlich, dass eine zentral von oben angeordnete optische Erfassung die wesentlichen Oberflächenbereiche beleuchtet und aufnehmen kann. Der variable Erfassungsbereich 8 kann somit entweder die gesamte Szene erfassen oder auch ausgewählte Teilbereiche. Über dem Bett 2 sind von oben angeordnet, ein optischer 3D-Sensor 5, eine Beleuchtungseinheit 10 und mindestens eine Videokamera 11. Wird die dreidimensionale Erfassung der Bettoberfläche nach dem Triangulationsprinzip mit strukturierter Beleuchtung realisiert, so kann hierzu die Videokamera mit herangezogen werden. Das gesamte System für eine dreidimensionale Erfassung und davon ausgelöster Videoüberwachung besteht aus einer Videokamera, einer flächenhaften Beleuchtung, sowie einer strukturierten Beleuchtung, die in einem bestimmten Winkel zur optischen Achse der Videokamera angeordnet ist.

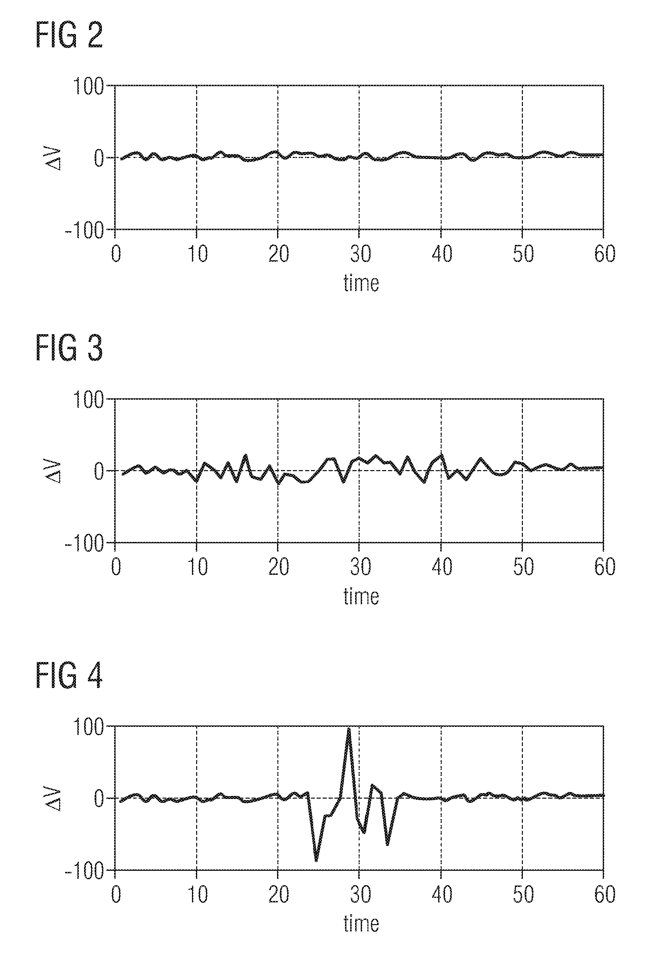

In den Figuren 2, 3 und 4 dargestellte 3D-Bewegungsanalysen betreffen jeweils in Figur 2 einen ruhig, nicht in Bewegung und nicht unruhig schlafenden Menschen. Der Graph entsprechend Figur 3 signalisiert einen unruhig liegenden Patienten, der sich regelmäßig bewegt.

Figur 4 zeigt eine erregte Person, die kräftige und kurzzeitige Bewegungen ausführt.

Für sämtliche Diagramme entsprechend den Figuren 2, 3 und 4 sind fortlaufende Beleuchtungen der Objektszene, Erfassungen der Objektszene mittels 3D-Sensorik und fortlaufende Berechnungen hinsichtlich der Bewegungsanalyse notwendig. Solange keine Grenzwerte überschritten werden, wird keine Videokamera zugeschaltet, die eine Bettszene aufgrund eines Ereignisses, das beispielsweise in Figur 4 dargestellt ist, bildlich an einen zentralen Videomonitor weiterleiten muss.

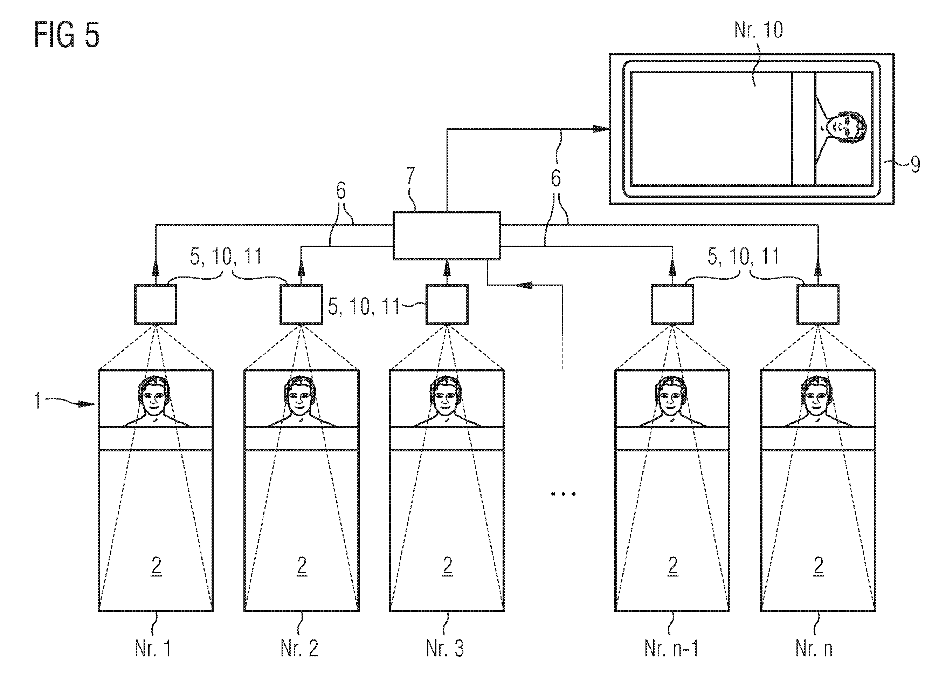

Figur 5 zeigt eine Vielzahl von Betten mit der Nummerierung 1 bis n, wobei jedes Bett mit einer Videokamera ausgestattet ist und prinzipiell Folgendes abläuft:

Mittels der Beleuchtungseinheit 10, die im Infrarotbereich arbeitet, wird jeweils ein Bett beleuchtet und mittels des optischen 3D-Sensors 5 erfasst. Ein Oberflächenbild wird in dreidimensionaler Form erzeugt, anhand dessen fortlaufende Bewegungsanalysen auf der Grundlage von Volumenänderungen einer Bettszene automatisch durchgeführt werden. Dies geschieht für jedes Bett einzeln in den entsprechenden Einheiten, die in der Regel einen optischen 3D-Sensor 5, eine Infrarot-Beleuchtungseinheit 10, und eine Videokamera 11 umfassen.

Wird durch zu häufige bzw. zu kräftige Bewegung eine deutliche Volumenänderung an einem bestimmten Bett aufgezeichnet, so erfolgt eine Überschreitung bestimmter vorgegebener Grenzwerte und eine Videokamera am entsprechenden Bett wird zur Aufnahme eines Videobildes zugeschaltet. Dieses Videobild wird über eine Auswerteeinheit 7 zu einem zentralen Videomonitor 9 weitergeleitet. In Figur 5 ist als Beispiel das Bett Nr. 10 als kritisch bei der Bewegungsanalyse bewertet, mittels der Videokamera 11 aufgenommen und durchgeschaltet worden.

Die Videoleitungen 6 können in vorteilhafter Weise durch Funkübertragung ersetzt werden.

Wesentlich ist, dass die Durchschaltung einer Videokamera zur Übermittlung eines Videobildes auf einen zentralen Beobachtungsmonitor auf der Basis einer dreidimensionalen Bewegungsanalyse im Bereich jeweils eines Patientenbettes ermittelt wird. Dazu werden jeweils die Betten einzeln mit den darin liegenden Patienten von oben mit einem optischen 3D-Sensor erfasst, der im nahen Infrarotbereich arbeitet.

## Patentansprüche

1. Verfahren zur zentralen Überwachung ruhender Personen bestehend aus folgenden Schritten:
- Fortlaufende Erfassung einer im infrarot beleuchteten oberen Oberfläche eines Bettes (2) mit darin ruhender Person (1) mittels eines dreidimensional auflösenden Sensors (5) und Ausgabe eines 3D-Signals,
- fortlaufende Berechnung des Bettvolumens und dessen zeitlicher Veränderung auf der Basis des 3D-Bilds,
- Bewegungsanalyse der überwachten Person, wobei aufgrund einer Überschreitung mindestens eines festgelegten Grenzwertes bezüglich der Volumenänderung mindestens eine über dem Bett angeordnete Videokamera (11) aktiviert wird und
- Signale der Videokamera (11) auf einen zentralen Videomonitor (9) geschaltet werden.

2. Verfahren nach Anspruch 1, bei dem mindestens ein vorgegebener Teilbereich der Oberfläche (3) eines Bettes (2) mit ruhender Person (1) erfasst und ausgewertet wird.

3. Verfahren nach einem der vorhergehenden Ansprüche, bei dem eine Videokamera (11) im nahen Infrarotbereich betrieben wird.

4. Verfahren nach einem der vorhergehenden Ansprüche, bei dem die Übertragung von Videobildern zu einem zentralen Videomonitor drahtlos erfolgt.

5. Verfahren nach einem der vorhergehenden Ansprüche, bei dem eine Bett-Identifizierung im Videobild übertragen wird.

6. Verfahren nach einem der vorhergehenden Ansprüche, bei dem eine dreidimensional auflösender Sensor entweder mit einer strukturierten Beleuchtung des Objektes und unterschiedlichen Richtungen für Beleuchtung und Erfassung zur Triangulation betrieben wird oder bei dem ein 3D-Sensor eingesetzt wird, der mittels Lichtlaufzeiten-Messung mit unstrukturierter Infrarotbeleuchtung funktioniert und dessen Beleuchtungs- und Erfassungsrichtung identisch sind.

7. Verfahren nach einem der vorhergehenden Ansprüche, bei dem eine einzige Videokamera sowohl als Aufnahmeeinheit für den 3D-Sensor als auch für die Aufnahme eines Videobildes zur Weiterleitung an einen zentralen Videomonitor in Kombination mit einer Lichtquelle für strukturierte Beleuchtung und einer Lichtquelle für eine unstrukturierte Beleuchtung eingesetzt wird.

8. Anordnung zur Aufnahme, Auswertung und selektiven Anzeige von ruhenden Personen aufgrund von aufgenommenen Bewegungsanalysen, welche Folgendes umfasst:
- eine Beleuchtungseinheit (10), die die obere Oberfläche eines Bettes mit darin ruhender Person im Infrarotbereich von oben vertikal oder schräg beleuchtet,
- einen über einem Bett mit darin ruhender Person beabstandet angeordneten optischen dreidimensional auflösenden Sensor (5) zur Generierung eines 3D-Bilds,
- eine Auswerteeinheit (7) zur Durchführung einer Bewegungsanalyse auf der Basis zeitlicher Veränderungen des Bettvolumens,
- einer über dem Bett (2) zentral oder schräg angeordneten Videokamera (11) in Kombination mit einer Beleuchtung im nahen Infrarotbereich zur Aufnahme eines Videobildes der Oberfläche des Bettes und zu dessen Weiterleitung an einen zentralen Videomonitor (9).

9. Anordnung nach Anspruch 8, bei der eine einzige Videokamera als optische Einheit sowohl für den 3D-Sensor zur Generierung eines 3D-Bilds eingesetzt wird, als auch als Videokamera zur Aufnahme eines herkömmlichen Bildes, welches an einen zentralen Videomonitor (9) übermittelbar ist.

10. Anordnung nach Anspruch 8, bei der ein dreidimensional erfassender Lichtlaufzeitsensor zur Generierung eines 3D-Bilds vorhanden ist und eine einzige Videokamera zur Aufnahme eines herkömmlichen Videobilds, welches an einen zentralen Videomonitor (9) übermittelbar ist.
